# EUROPEAN PATENT APPLICATION

(11) **EP 4 193 938 A1**
(43) Date of publication of application: **14.06.2023**
(21) Application number: 22212004.0
(22) Date of filing: 07.12.2022
(51) Int. Cl.: A61B 17/22, A61B 17/00

(54) **SYSTEMS FOR SEPARATING NATIVE HEART VALVE LEAFLETS ATTACHED TOGETHER BY A FIXATION DEVICE**

(30) Priority: 09.12.2021 US 202163287897 P
(71) Applicant: Evalve, Inc., Santa Clara, CA 95054 (US)
(72) Inventor: WEI, Michael F, Redwood City, 94065 (US)
(74) Representative: Boult Wade Tennant LLP

(57) **Abstract**

Systems and methods for separating native heart valve leaflets attached together by a fixation device. Systems including an elongate shaft having a proximal end portion, a distal end portion and a longitudinal axis extending therebetween, the elongate shaft configured for transvascular delivery of the distal end portion to a native heart valve. At least one arm extending from the distal end portion, and a cutter disposed along at least one of the distal end portion and the at least one arm. The at least one arm is configured to extend through an orifice defined between native heart valve leaflets attached together by a fixation device to position the cutter against one of the native heart valve leaflets adjacent the fixation device, the cutter configured to cut through native heart valve leaflet tissue.

## Description

### CROSS-REFERENCE TO RELATED APPLICATIONS

This application claims priority to U.S. Provisional Application No. 63/287,897 filed December 9, 2021, the disclosures of which are hereby expressly incorporated by reference in their entirety.

### BACKGROUND

Mitral valve regurgitation is characterized by retrograde flow from the left ventricle of a heart through an incompetent mitral valve into the left atrium. During a normal cycle of heart contraction (systole), the mitral valve acts as a check valve to prevent flow of oxygenated blood back into the left atrium. In this way, the oxygenated blood is pumped into the aorta through the aortic valve. Regurgitation of the valve can significantly decrease the pumping efficiency of the heart, placing the patient at risk of severe, progressive heart failure.

Mitral valve regurgitation can result from a number of different mechanical defects in the mitral valve or the left ventricular wall. The valve leaflets, the valve chordae which connect the leaflets to the papillary muscles, the papillary muscles themselves or the left ventricular wall may be damaged or otherwise dysfunctional. Commonly, the valve annulus can be damaged, dilated, or weakened, limiting the ability of the mitral valve to close adequately against the high pressures of the left ventricle.

The most common treatments for mitral valve regurgitation rely on valve replacement or repair including leaflet and annulus remodeling, the latter generally referred to as valve annuloplasty. One technique for mitral valve repair which relies on suturing adjacent segments of the opposed valve leaflets together is referred to as the "bow-tie" or "edge-to-edge" technique. While all these techniques can be effective, they often rely on open heart surgery where the patient's chest is opened, frequently via a sternotomy, with the patient placed on cardiopulmonary bypass. The need to both open the chest and place the patient on bypass can be traumatic.

In some patients, a fixation device can be installed into the heart using minimally invasive techniques. The fixation device can hold the adjacent segments of the opposed valve leaflets together and may reduce mitral valve regurgitation. One such device used to clip the anterior and posterior leaflets of the mitral valve together is the MitraClip^{®} fixation device, sold by Abbott Vascular, Santa Clara, Calif., USA.

Sometimes after a fixation device is installed mitral valve regurgitation can still exist, or can arise again. Further, other problems with the heart may arise that can make it desirable for the fixation device to be disabled or removed. For example, it can be desirable to remove the fixation device to allow for implantation of a replacement heart valve. For at least these reasons, it is desirable to provide systems and methods for removing or disabling fixation devices that are already installed.

### SUMMARY

The purpose and advantages of the disclosed subject matter will be set forth in and apparent from the description that follows, as well as will be learned by practice of the disclosed subject matter. For purpose of illustration and not limitation, the various embodiments described herein relate to systems and methods for separating native heart valve leaflets attached together by a fixation device. Additional advantages of the disclosed subject matter will be realized and attained by the systems and methods particularly pointed out in the written description and claims hereof, as well as from the appended drawings.

To achieve these and other advantages, and in accordance with the purpose of the disclosed subject matter, as embodied and broadly described, the disclosed subject matter includes systems for separating native heart valve leaflets attached together by a fixation device. Systems in accordance with the disclosed subject matter include an elongate shaft including a proximal end portion, a distal end portion and a longitudinal axis extending therebetween. The elongate shaft is configured for transvascular delivery of the distal end portion to a native heart valve. At least one arm extends from the distal end portion, and a cutter is disposed along at least one of the distal end portion and the at least one arm. The at least one arm is configured to extend through an orifice defined between native heart valve leaflets attached together by a fixation device to position the cutter against one of the native heart valve leaflets adjacent the fixation device. The cutter is configured to cut through native heart valve leaflet tissue.

The elongate shaft can include a steering mechanism adapted to bend at least a portion of the elongate shaft in at least a first reference plane. Additionally or alternatively, the steering mechanism can be adapted to bend at least a portion of the elongate shaft in a second reference plane, the second reference plane being generally perpendicular to the first reference plane. The steering mechanism can include at least one cable extending the length of the elongate shaft.

Additionally or alternatively, the at least one arm can include an arm distal end portion and an arm length extending between the distal end portion and the arm distal end portion, the at least one arm can be deployable between a delivery condition and a cutting condition, the at least one arm in the delivery condition can extend along the longitudinal axis, and the at least one arm in the cutting condition can extend radially outward from the longitudinal axis and the distal end portion to define a hook, the at least one arm can be configured to be positioned around one of the native heart valve leaflets with the distal end portion and the arm distal end portion extending through opposing orifices on either side thereof defined between the native heart valve leaflets attached together by a fixation device.

Additionally or alternatively, the cutter can extend between the distal end portion and the arm distal end portion.

Additionally or alternatively, a slot can be defined in an outer wall of the at least one arm, the slot extending between the distal end portion and the arm distal end portion, and the cutter can be configured to be deployed from the slot to extend away from an apex of the hook in the cutting condition.

Additionally or alternatively, a cutter height can be defined between the cutter and the apex of the hook in the cutting condition. For example and not limitation, the cutter height can be between approximately 5mm and approximately 10mm.

Additionally or alternatively, the cutter can be radially offset from the at least one cable when the elongate shaft is viewed in transverse cross section.

Additionally or alternatively, the cutter can include a wire.

Additionally or alternatively, the cutter can be affixed to the arm distal end portion, and the cutter can be configured to transition the at least one arm from the delivery condition to the cutting condition upon application of tension thereto.

Additionally or alternatively, a hook width can be defined across the hook between the distal portion and arm distal end portion in the cutting condition. For example and not limitation, the hook width can be between approximately 5mm and approximately 15mm.

Additionally or alternatively, the cutter can include a conductive pad extending along the at least one arm. Additionally or alternatively, the conductive pad can be adhered to an outer wall of the at least one arm.

Additionally or alternatively, the cutter can be conductive and can be configured to be energized with RF energy to cut through native heart valve leaflet tissue. The RF energy can be monopolar. Additionally or alternatively, the RF energy can be bipolar and the system can include a current return path.

Additionally or alternatively, the distal end portion can include at least one lumen defined therein, and the at least one arm can be deployable from the lumen between a retracted position and an extended position.

Additionally or alternatively, the at least on arm can comprise one or more of stainless steel, and nitinol.

Additionally or alternatively, the at least one arm in the extended position can defines a hook, and the hook can be configured to be positioned around one of the native heart valve leaflets and to draw the native heart valve leaflet against the cutter as the at least one arm is retracted towards the retracted position to cut through native heart valve leaflet tissue.

Additionally or alternatively, the cutter can be disposed at the distal portion of the elongate shaft. Additionally or alternatively, the cutter can comprise a blade. Additionally or alternatively, the at least one arm can include the cutter.

Additionally or alternatively, the at least one arm can include two opposing arms.

Additionally or alternatively, the opposing arms can be configured to spread away from one another as the opposing arms deploy from the retracted position toward the extended position. The opposing arms in the extended position can be configured to extend through opposing orifices on either side of one of the native heart valve leaflets defined between the native heart valve leaflets attached together by a fixation device. The opposing arms can be configured to approach one another as the opposing arms are retracted towards the retracted position to trap the native heart valve leaflet therebetween.

Additionally or alternatively, opposing arm distal ends of the opposing arms in the extended position can define an opposing arm distance therebetween. For example and not limitation, the opposing arm distance can be between approximately 5mm and approximately 15mm.

Additionally or alternatively, the cutter can include a wire extending between the opposing arms. For example, the wire can extend between respective opposing arm distal ends.

Additionally or alternatively, the opposing arms can be rigid.

Additionally or alternatively, the cutter can be disposed along at least one of the opposing arms.

The disclosed subject matter further includes methods for separating native heart valve leaflets attached together by a fixation device. Methods in accordance with the disclosed subject matter include delivering a distal end portion of a system for separating native heart valve leaflets attached together by a fixation device as described above to a native heart valve. Methods in accordance with the disclosed subject matter further include extending the at least one arm through an orifice defined between native heart valve leaflets attached together by a fixation device and positioning the cutter against one of the native heart valve leaflets adjacent the fixation device. Methods in accordance with the disclosed subject matter further include cutting the native heart valve leaflet with the cutter to separate the native heart valve leaflets.

It is to be understood that both the foregoing general description and the following detailed description are exemplary and are intended to provide further explanation of the disclosed subject matter claimed.

The accompanying drawings, which are incorporated in and constitute part of this specification, are included to illustrate and provide a further understanding of the systems and methods of the disclosed subject matter. Together with the description, the drawings serve to explain the principles of the disclosed subject matter.

### BRIEF DESCRIPTION OF THE DRAWINGS

FIG. 1 illustrates the left ventricle and left atrium of the heart during systole.
FIG. 2. illustrates an exemplary fixation device clipping together the anterior and posterior leaflets of a mitral valve.
FIG. 3A illustrates a cross-sectional side view of a mitral valve having a fixation device clipping together the anterior and posterior leaflets of the mitral valve.
FIG. 3B illustrates a cross-sectional side view of the mitral valve of FIG. 3A after separation of the anterior and posterior leaflets with the fixation device remaining clipped to the posterior leaflet.
FIG. 4 is a schematic view depicting an exemplary elongate shaft suitable for use in a system in accordance with the disclosed subject matter and various positions of the distal end portion of the elongate shaft;
FIG. 5A is a top plan view of an exemplary system for separating native heart valve leaflets in accordance with the disclosed subject matter, depicting the at least one arm in a delivery condition.
FIG. 5B is a perspective view of the system of FIG. 5A, depicting the at least one arm in a cutting condition.
FIG. 6 is a transverse cross-sectional view of the system of FIG. 5A taken along line 6-6 as shown in FIG. 5A.
FIG. 7A is a top plan view of another exemplary system for separating native heart valve leaflets in accordance with the disclosed subject matter, depicting the at least one arm in a delivery condition.
FIG. 7B is a perspective view of the system of FIG. 7A, depicting the at least one arm in a cutting condition.
FIG. 8A is a top plan view of another exemplary system for separating native heart valve leaflets in accordance with the disclosed subject matter, depicting the at least one arm in a delivery condition.
FIG. 8B is a perspective view of the system of FIG. 8A, depicting the at least one arm in a cutting condition.
FIG. 9 is a cross-sectional view of a native mitral valve taken along the native mitral valve annulus looking down from the left atrium. The native mitral valve has a fixation device clipping together the native heart valve leaflets, and an exemplary system for separating native heart valve leaflets in accordance with the disclosed subject matter is positioned within the native mitral valve with the at least one arm positioned around one of the native heart valve leaflets in a cutting condition.
FIG. 10A is a top plan view of another exemplary system for separating native heart valve leaflets in accordance with the disclosed subject matter, depicting the arms deploying to an extended position.
FIG. 10B is a top plan view of the system of FIG. 10A, depicting the arms retracting towards a retracted position.
FIG. 11A is a front cross-sectional view of a native mitral valve. The native mitral valve has a fixation device clipping together the native heart valve leaflets, and the system of FIG. 10A is positioned at the native mitral valve with arms extending through orifices defined on either side of the native mitral valve leaflets.
FIB. 11B is the front cross-sectional view of FIG. 11A depicting the system of FIG. 10A with the arms retracting to trap a native mitral valve leaflet therebetween.
FIG. 12A is a top plan view of another exemplary system for separating native heart valve leaflets in accordance with the disclosed subject matter, depicting arms deploying to an extended position.
FIG. 12B is a top plan view of the system of FIG. 12A, depicting the arms further deploying to an extended position.
FIG. 12C is a front cross-sectional view of a native mitral valve. The native mitral valve has fixation devices clipping together the native heart valve leaflets, and the system of FIG. 12A is extending through orifices defined between the native heart valve leaflets to position the cutter against one of the native heart valve leaflets adjacent the fixation device.
FIG. 13 is a top plan view of another exemplary system for separating native heart valve leaflets in accordance with the disclosed subject matter.
FIG. 14 is a top plan view of another exemplary system for separating native heart valve leaflets in accordance with the disclosed subject matter.

### DETAILED DESCRIPTION

Reference will now be made in detail to the various exemplary embodiments of the disclosed subject matter, which are illustrated in the accompanying drawings. The structure and corresponding method of operation of the disclosed subject matter will be described in conjunction with the detailed description of the system. The accompanying drawings, where like reference numerals refer to identical or functionally similar elements throughout the separate views, serve to further illustrate various embodiments and to explain various principles and advantages all in accordance with the disclosed subject matter.

The disclosed subject matter is directed to systems and methods for separating native heart valve leaflets attached together by a fixation device. Although embodiments described herein are directed to separating native mitral valve leaflets attached together by a fixation device, it will be understood that systems and methods in accordance with the disclosed subject matter can also be applied to separate leaflets of other heart valves attached together by a fixation device, such as the tricuspid valve, aortic valve, or pulmonary valve. Additionally, the term fixation device as used herein encompasses both the singular-fixation device-as well as a plurality of fixation devices. That is, it is understood that one or more fixation devices may be used to attach native heart valve leaflets together, and systems and methods in accordance with the disclosed subject matter can be used to separate the native heart valve leaflets attached together by the one or more fixation devices.

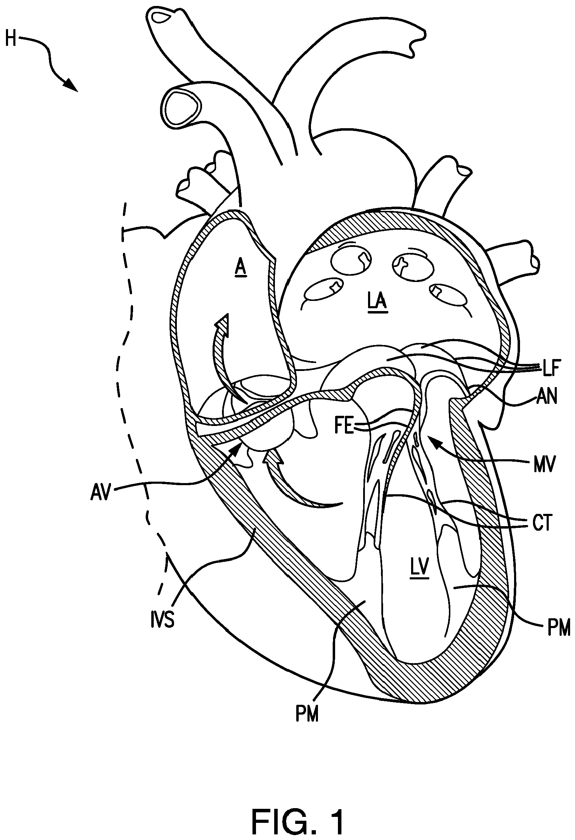

The left ventricle (LV) of a normal heart H in systole is illustrated in FIG. 1. The left ventricle (LV) is contracting and blood flows outwardly through the tricuspid (aortic) valve (AV) in the direction of the arrows. Back flow of blood or "regurgitation" through the mitral valve (MV) is prevented since the mitral valve is configured as a "check valve" which prevents back flow when pressure in the left ventricle is higher than that in the left atrium (LA). The mitral valve (MV) comprises a pair of leaflets having free edges (FE) which meet evenly to close, as illustrated in FIG. 1. The opposite ends of the leaflets (LF) are attached to the surrounding heart structure along an annular region referred to as the annulus (AN). The free edges (FE) of the leaflets (LF) are secured to the lower portions of the left ventricle LV through chordae tendinae (CT) which include a plurality of branching tendons secured over the lower surfaces of each of the valve leaflets (LF). The chordae (CT) in turn, are attached to the papillary muscles (PM) which extend upwardly from the lower portions of the left ventricle and intraventricular septum IVS.

A number of structural defects in the heart can cause mitral valve regurgitation. Regurgitation occurs when the valve leaflets do not close properly allowing leakage from the ventricle into the atrium. For example, enlargement of the heart can cause the mitral annulus to become enlarged such that the free edges (FE) of the mitral valve do not meet during systole. This can result in a gap which allows blood to leak through the valve during ventricular systole. Ruptured or elongated chordae tendinae can also cause one or more valve leaflets to prolapse such that the two valve leaflets do not properly meet and leakage occurs from the left ventricle into the left atrium. Such regurgitation can also occur in patients who have suffered ischemic heart disease where the left ventricle does not contract sufficiently to effect proper closure.

Fixation devices can be used for grasping, approximating and fixating tissues such as valve leaflets to treat cardiac valve regurgitation, particularly mitral valve regurgitation. Additional examples and details related to fixation devices, delivery devices for directing fixation devices to a targeted treatment area, handles, and deployment mechanisms, are described in U.S. Patent Number 7,666,204, U.S. Patent Number 7,563,267, and U.S. Patent Application Publication No. 2017/0100250, the disclosures of each of which are incorporated herein in their entirety by this reference. One such device used to clip the anterior and posterior leaflets of the mitral valve together is the MitraClip^{®} fixation device, sold by Abbott Vascular, Santa Clara, Calif., USA.

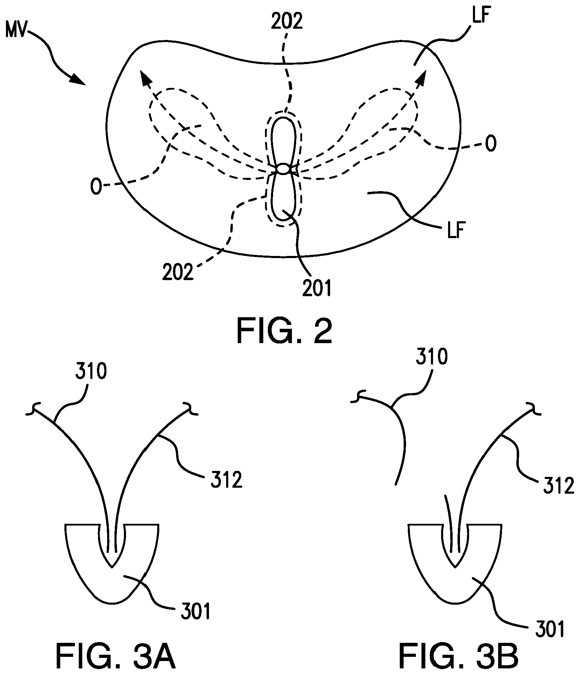

FIG. 2 illustrates an exemplary fixation device clipping together the anterior and posterior leaflets of a mitral valve. FIG. 2 is a taken from the atrial side of the mitral valve (MV), and therefore, the atrial elements 201 of the fixation device are shown in solid line and the ventricular elements 202 are shown in dashed line for purpose of illustration and understanding. The leaflets LF are held in place so that during diastole, as shown in FIG. 2, the leaflets LF remain in position between the atrial elements 201 and ventricular elements 202 surrounded by openings or orifices O which result from the diastolic pressure gradient. As depicted in FIG. 2, the leaflets LF can be coapted or attached together such that their upstream surfaces are facing each other in a vertical orientation, parallel to the direction of blood flow through mitral valve MV.

FIG. 3A illustrates a side cross-sectional view of a mitral valve having native leaflets attached together by a fixation device 301. As embodied herein, the fixation device 301 can be coupled to the anterior leaflet 310 and posterior leaflet 312. As described further herein, separating the native leaflets may be desirable to facilitate further treatment of the native mitral valve, such as by installing a replacement heart valve, or to satisfy another clinical need. However, fixation devices can be embedded with the leaflet tissue and/or other surrounding tissues as a result of tissue ingrowth, making it difficult to extract the implant and/or separate the native leaflets. With reference to FIG. 3B, separation of the native leaflets can be achieved by tearing, cutting, or severing the tissue of one or more of the leaflets. For example, and as embodied herein, separation can be achieved by tearing the native mitral valve anterior leaflet tissue with the fixation device 301 remaining attached to the posterior leaflet 312 after the leaflets are separated. As described further herein, tearing of the anterior leaflet tissue can be advantageous, as there is less risk that the fixation device will interfere with functioning of the left ventricular outflow tract when the fixation device remains attached to the posterior leaflet.

Systems for separating native heart valve leaflets attached together by a fixation device in accordance with the disclosed subject matter include an elongate shaft including a proximal end portion, a distal end portion and a longitudinal axis extending therebetween. The elongate shaft is configured for transvascular delivery of the distal end portion to a native heart valve. At least one arm extends from the distal end portion, and a cutter is disposed along at least one of the distal end portion and the at least one arm. The at least one arm is configured to extend through an orifice defined between native heart valve leaflets attached together by a fixation device to position the cutter against one of the native heart valve leaflets adjacent the fixation device. The cutter is configured to cut through native heart valve leaflet tissue.

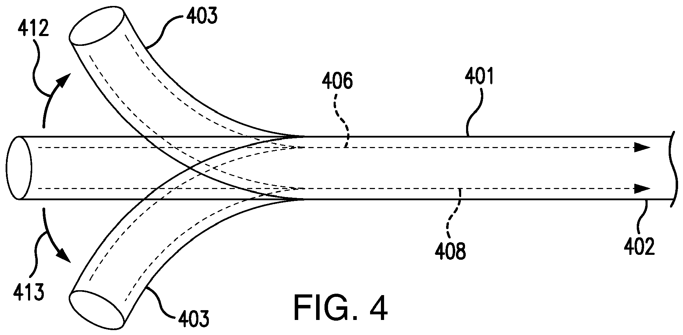

FIG. 4 is a schematic view depicting an exemplary elongate shaft 401 suitable for use in a system in accordance with the disclosed subject matter. The elongate shaft includes a proximal end portion 402 and a distal end portion 403 and a longitudinal axis extending therebetween. Techniques for delivering elongate shafts to a native heart valve are known in the art. For example, the elongate shaft can be delivered to the native heart valve transapically. Alternatively, and as embodied herein, the elongate shaft 401 can be delivered to the native heart valve transvascularly. For example, the elongate shaft 401 can be delivered to the native heart valve using a transseptal approach. In transseptal applications, the elongate shaft 401 can be used in in conjunction with a transseptal sheath or introducer catheter or independently passed through the septum. Additionally or alternatively, the elongate shaft 401 can include an atraumatic blunt or tapered tip to traverse through vasculature safely.

As embodied herein, the elongate shaft 401 can be a steerable catheter. The construction of steerable catheters is known in the art. For example, the elongate shaft can include a laminate, or composite, construction of flexible polymers, such as Pebax or Nylon. As is known in the art, stainless steel braiding can be incorporated in the elongate shaft construction to control stiffness and hoop strength of the elongate shaft. Additionally or alternatively, the elongate shaft 401 can incorporate a hydrophilic coating for lubricity. The dimensions and construction of the elongate shaft 401 can be selected to achieve the desired performance of the system and to access the native heart valve using the desired surgical technique. As described further herein, the diameter of the elongate shaft can be selected to position a cutter against one of the native heart valve leaflets at a desired distance from a previously implanted fixation device. Furthermore, the construction of the elongate shaft can be selected to facilitate application of the desired tension to a cutter as described further herein.

The elongate shaft 401 can include steering mechanisms to position the distal end portion 403 of the elongate shaft 401. In accordance with an aspect of the disclosed subject matter, the steering mechanism can include at least one cable extending along a length of the elongate shaft. For purpose of example, and as embodied herein, the steering mechanism can include a plurality of cables 406, 408 extending a length of the elongate shaft 401. As shown, elongate shaft 401 can include a first cable 406 slidably disposed in a lumen within the wall of the elongate shaft 401 and extending a length of the elongate shaft to the distal end portion 403. By applying tension to the cable 406 in the proximal direction, the distal end portion 403 curves in the direction of the cable 406 as illustrated by arrow 412. Likewise, placement of a second cable 408 along the opposite side of the elongate shaft 401 can allow the distal end portion 403 to be curved in the opposite direction, as illustrated by arrow 414, when tension is applied to the second cable 408.

Thus, the opposed cables 406 and 408 within the walls of the elongate shaft 401 can enable the distal end portion 403 to be steered or bent in opposite directions. As embodied herein, the steering mechanism can include a delivery handle (not shown) having one or more steering knobs for controlling the tensioning of one or more of the cables 406, 408 running the length of the elongate shaft 401. This can provide a means of correcting or adjusting a curvature of the elongate shaft 401 within one or more reference planes, as described further herein. For example, if tension is applied to one cable to create a curvature, the curvature can be lessened by applying tension to the diametrically opposite cable. The illustrated embodiment includes two opposing cables. Other embodiments can include a single cable, or can include more than two cables. In addition, cables and associated lumens can be placed in any arrangement, singly or in pairs, symmetrically or non-symmetrically, to enable desired curvature capabilities. Cables can be fixed at any location along the length of the elongate shaft 401 by any suitable method, such as gluing, tying, soldering, and the like. Additionally or alternatively, cables can extend through the distal end portion of the elongate shaft 401 and be fixed at a location along the at least one arm, as further described herein. When tension is applied to the cable, the curvature forms from the point of attachment of the cable toward the proximal direction. Additionally, or alternatively, the elongate shaft 401 can be precurved to provide a desired angling for properly traversing a patient's vasculature in the context of a particular procedural approach.

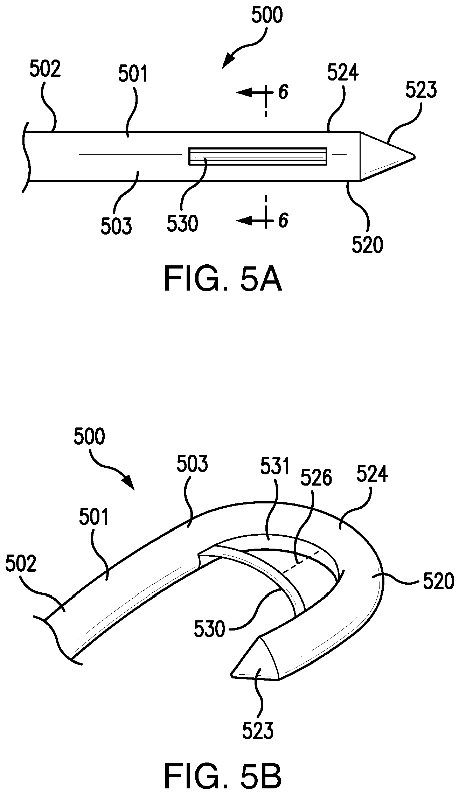

FIGs. 5A and 5B depict an exemplary system 500 for separating native heart valve leaflets in accordance with the disclosed subject matter. The system 500 includes an elongate shaft 501 having a proximal end portion 502, a distal end portion 503 and a longitudinal axis extending therebetween. The elongate shaft 501 is configured for transvascular delivery of the distal end portion 503 to a native heart valve. As described above, the elongate shaft 501 can be a steerable catheter. In accordance with the disclosed subject matter, the system 500 includes at least one arm 520 extending from the distal end portion 503, and a cutter 530 is disposed along at least one of the distal end portion 503 and the at least one arm 520. As described further herein, the at least one arm 520 is configured to extend through an orifice defined between native heart valve leaflets attached together by a fixation device to position the cutter 530 against one of the native heart valve leaflets adjacent the fixation device, and the cutter 530 is configured to cut through native heart valve leaflet tissue.

In accordance with an aspect of the disclosed subject matter, the arm 520 can include an arm distal end portion 523, and an arm length 524 extending between the distal end portion 503 of the elongate shaft 501 and the arm distal end portion 523. The arm 520 can be deployable between a delivery condition and a cutting condition. With reference to FIG. 5A, the arm 520 is depicted in a delivery condition with the arm 520 extending along the longitudinal axis. Having the arm 520 aligned with the elongate shaft 501 along the longitudinal access in the delivery condition can be desirable to facilitate navigation of the system 500 through a patient's vasculature for delivery to the native heart valve. With reference to FIG. 5B, the arm 520 is depicted in a cutting condition with the arm distal end portion 523 extending radially outward from the longitudinal axis and the distal end portion 503 to define a hook. In accordance with an aspect of the disclosed subject matter, the arm 520 can be configured to be positioned around one of the native heart valve leaflets with the distal end portion 503 and the arm distal end portion 523 extending through opposing orifices on either side thereof defined between the native heart valve leaflets attached together by a fixation device.

As embodied herein, a hook width can be defined between the distal portion 503 and arm distal end portion 523 with the arm 520 in the cutting condition. The hook width can be selected based on the type of native heart valve to be separated, and/or based on the anatomical measurements of a particular patient. The dimensions of the hook width can be pre-set, and offered in a range of widths to be selected from, to capture the target leaflet (e.g. anterior or posterior) to be enveloped and cut. A desired hook width can be selected by, for example, pre-procedural planning of the targeted leaflet to cut, including consideration of the relative thickness of the leaflet, degree of tissue encapsulation of the implant, or relative patient or anatomical considerations. For purpose of example, the hook width can be between approximately 5mm-15mm.

As embodied herein, a hook depth can be defined between the arm distal end portion 523 and the apex 528 of the hook. The dimensions of the elongate shaft and the distal end portion 523 can be selected to achieve a desired hook depth. For example, the hook depth can be selected to allow ease of orienting rotation and positioning of the hook, such as with limited imaging guidance (e.g., echo or fluoro), to move into the correct orifice and adjacent to the implant to remove, and/or to remain secure under mild tension. For purpose of example and not limitation, the hook depth can be between approximately 5mm-15mm.

As further embodied herein, the shape of the hook can vary along the hook. For example, the hook width defined between the distal-most portion of distal end portion 523 and distal portion 503 can be larger than the hook width defined between a more proximal portion of distal end portion 523 and distal portion 503 to define a flared or funnel-shaped hook. A flared or funnel-shaped hook can be desirable, for example, to facilitate funneling the target leaflet into the hook.

As described further herein, elongate shaft 501 can be retracted with the hook positioned around a native heart valve to cut a native heart valve leaflet. For example, RF energy can be applied to cutter 530 and elongate shaft 501 can be retracted with the hook under continuous tension to cut the native heart valve leaflet. As described further herein, RF energy can be applied to cutter 530 and the elongate shaft 501 can be retracted to sever a portion of the native heart valve leaflet and free an implanted fixation device attached thereto. As described further herein, the cutter 530 can be an electrode and can be statically affixed or exposed on the inner surface of the apex 528 of the hook and can cut tissue when energized with RF. Additionally or alternatively, the cutter 530 can include an internalized wire electrode and can be tensioned and presented at a different distance from the apex 528 of the hook to present a fully exposed RF electrode wire for leaflet cutting.

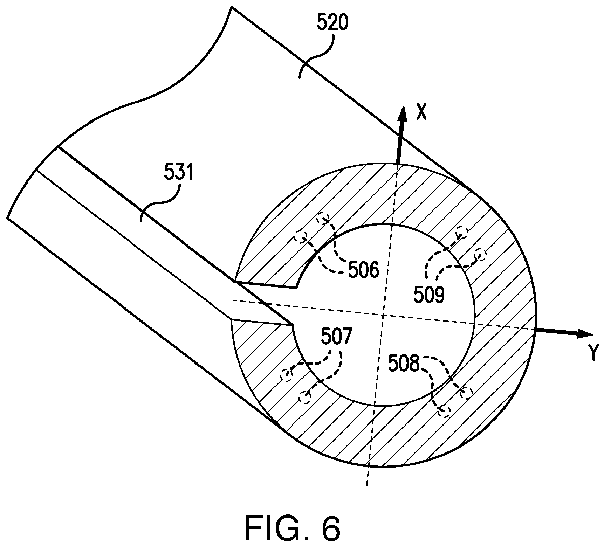

As described above, the elongate shaft 501 can include one or more cables 506, 507, 508, and 509 disposed in lumens within the wall of the elongate shaft 501 and the cables 506, 507, 508, and 509 can be used to control curvature of the elongate shaft 501. As embodied herein, cables 506, 507, 508, and 509 can extend through the distal end portion 503 and terminate in the arm 520 such that application of tension to the cables 506, 507, 508, and 509 can be used to deploy the arm 520 from the delivery condition to the cutting condition, the arm 520 defining a hook in the cutting condition. For purpose of example and as embodied herein, the cables 506, 507, 508, and 509 can be oriented around the circumference of the elongate shaft 501 and arm 520 such that the cables 506, 507, 508, and 509 are offset from curve planes X and Y as depicted in FIG. 6. For example, application of tension to cables 506 and 507 can cause the arm 520 to bend in reference plane Y to deploy the arm 520 from the delivery condition to the cutting condition. Likewise, application of tension to cable pairs 506 and 509 or 507 and 508 can cause the arm 520 to bend in reference plane X, generally perpendicular to reference plane Y. The ability to control movement of the distal end portion 503 and/or arm 520 can facilitate accurate positioning of the cutter 530 against one of the native heart valve leaflets, as described further herein. Additional degrees of directional control for the system 500 can be provided by, for example, a steerable outer guide catheter as described in U.S. Patent No. 7,666,204, the content of which is hereby incorporated by reference in its entirety.

As described above, the system 500 includes a cutter 530 disposed along at least one of the distal end portion 503 and the at least one arm 520. As embodied herein, a slot 531 can be defined in an outer wall of the at least one arm 520, and the slot 531 can extend between the distal end portion 503 and the arm distal end portion 523. The cutter 530 can extend between the distal end portion 503 and the arm distal end portion 523 in the slot 531 when the system 500 is in the delivery condition. Having the cutter 530 disposed in the slot 531 can be advantageous for effectively navigating the system 500 through a patient's vasculature for delivery to the native heart valve. With reference to FIG. 5B, the cutter 530 can be deployed from the slot to extend away from the apex 528 of the hook in the cutting condition. A cutter height 526 can be defined between the cutter 530 and an apex 528 of the hook in the cutting condition. The cutter height can be between approximately 1mm and 5mm, or approximately the radius of curvature of the hook.

In accordance with an aspect of the disclosed subject matter, the cutter 530 can be radially offset from the at least one cable when the elongate shaft is viewed in transverse cross section. As embodied herein the cutter 530 and slot 531 can be radially offset from the plurality of cables 506, 507 when the elongate shaft 501 is viewed in transverse cross-section. For example, the cutter 530 and slot 531 can extend along a centerline of the curve plane Y with cables 506 and 507 extending along either side thereof as depicted in FIG. 6. With the cutter 530 and slot 531 radially offset from the cables, slot 531 can be defined in the outer wall of the elongate shaft without interfering with the lumens defined in the elongate shaft for cables 506 and 507.

As described above, cables 506, 507, 508, and 509 can be used to control movement of the distal end portion 503 and/or arm 520. Additionally or alternatively, and as described further herein, application of tension to the cutter 530 can be used to control movement of the arm 520. For example, application of tension to the cutter 530 can be used to deploy the arm 520 from the delivery condition to the cutting condition. The cutter and one or more of cables 506, 507, 508, and 509 can be used independently or in combination to deploy the arm and control positioning of the system 500.

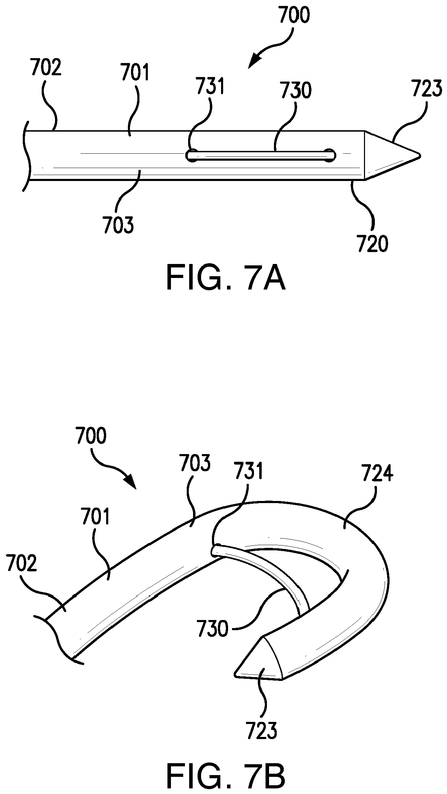

In accordance with another aspect of the disclosed subject matter, and with reference to the exemplary system 700 depicted in FIGs. 7A and 7B, the cutter 730 can extend between the distal end portion 703 and the arm distal end portion 723 along an exterior surface of the arm 720 in the delivery condition. In accordance with the disclosed subject matter, exemplary system 700 includes an elongate shaft 701 having a proximal end portion 702, a distal end portion 703 and a longitudinal axis extending therebetween. The elongate shaft 701 is configured for transvascular delivery of the distal end portion 503 to a native heart valve. Arm 720 extends from the distal end portion 703 of the elongate shaft, and a cutter 730 is disposed along at least one of the distal end portion 703 and the arm 720. As described further herein, the at least one arm 720 is configured to extend through an orifice defined between native heart valve leaflets attached together by a fixation device to position the cutter 730 against one of the native heart valve leaflets adjacent the fixation device, and the cutter 730 is configured to cut through native heart valve leaflet tissue.

With reference to FIG. 7A, exemplary system 700 is depicted in a delivery condition with the cutter 730 extending between the distal end portion 703 and the arm distal end portion 723 along an exterior surface of the arm 720. As embodied herein, the elongate shaft can include a lumen defined therein and an opening 731 to the lumen can be defined in the distal end portion 703 of the elongate shaft 701. The cutter 703 can extend from the opening 731 along an exterior surface of the arm 720 and the distal end of the cutter 730 can be affixed to the arm distal end portion 723. Tension can be applied to the cutter 730 to control the position of the arm distal end portion 723 as described above. For example, tension can be applied to the cutter from the proximal end 702 of the elongate shaft 701 to transition the arm 720 from the delivery condition to the cutting condition.

With reference to FIG. 7B, the exemplary system 700 is depicted in a cutting condition with the arm distal end portion 723 extending radially outward from the longitudinal axis and the distal end portion 703 to define a hook. As described further herein, the arm 520 is configured to be positioned around a native heart valve leaflet with the distal end portion 503 and the arm distal end portion 523 extending through opposing orifices on either side thereof defined between the native heart valve leaflets attached together by a fixation device.

In accordance with the disclosed subject matter, the cutter 730 is configured to cut through native heart valve leaflet tissue. The cutter can have any suitable configuration for cutting through native heart valve leaflet tissue. For example, the cutter can be comprised of a stainless steel wire. Additionally or alternatively, the cutter 730 can be conductive material, such as a conductive wire, strip, or line, and can be energized to cut through native heart valve leaflet tissue. For example, and as embodied herein, the cutter 730 can comprise a conductive metal wire and RF energy can be transmitted along the wire to cut native heart valve leaflet tissue. For example, the cutter 730 can extend along an insulated lumen within the elongate shaft 701 to the proximal end 702, and a RF generator console I/O connection can be used to transmit RF energy along the cutter 730. The portion of the cutter 730 extending between the distal end portion 703 and the arm distal end portion 723 can be exposed, or uninsulated, which can facilitate application of RF energy from the cutter 730 to native heart valve leaflet tissue. As embodied herein the monopolar RF energy can be used in conjunction with a grounding pad to the patient. Alternatively, bipolar RF energy can be used with a return conductive path. A set of parallel conductors can be isolated by a distance between them, where each conductor is of an opposite polarity. Alternatively, one conductor can be positive and the other conductor can be ground. Once a charge is applied to one conductor, or each having an opposite polarity charge, an arch can cross from one to the next and cause a cautery effect resulting in focused cut when conductive tissue is next to these charged conductors.

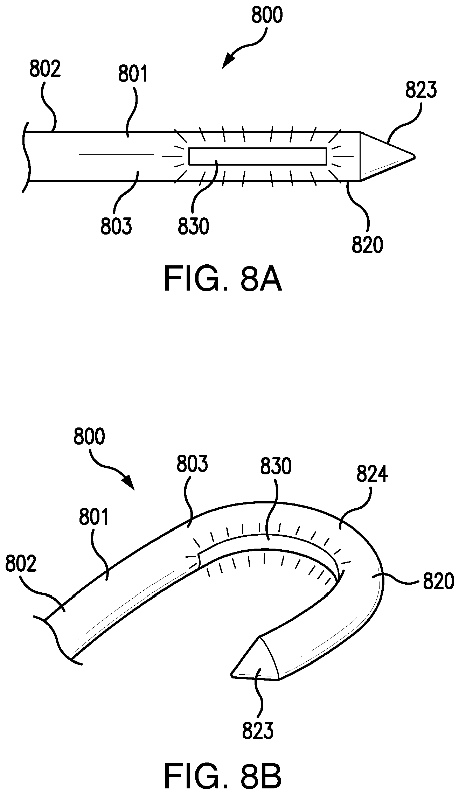

In accordance with an aspect of the disclosed subject matter, and with reference to the exemplary system 800 depicted in FIGs. 8A and 8B, the cutter 830 can include a conductive pad extending along the at least one arm 820. Exemplary system 800 includes an elongate shaft 801 having a proximal end portion 802, a distal end portion 803 and a longitudinal axis extending therebetween. The elongate shaft 801 is configured for transvascular delivery of the distal end portion 503 to a native heart valve. Arm 820 extends from the distal end portion 803 of the elongate shaft, and a cutter 830 is disposed along at least one of the distal end portion 803 and the arm 820. As embodied herein the cutter 830 includes a conductive pad configured to cut through native heart valve leaflet tissue. The conductive pad can be configured to be energized with monopolar or bipolar RF energy to cut through native heart valve leaflet tissue, as described above.

As described above and as embodied herein, the arm 820 can include an arm distal end portion 823, and an arm length 824 extending between the distal end portion 803 of the elongate shaft 801 and the arm distal end portion 823. The arm 820 can be deployable between a delivery condition and a cutting condition. With reference to FIG. 8A, the arm 820 is depicted in a delivery condition with the arm 820 extending along the longitudinal axis. The distal end portion 823 of the arm 820 can have a tapered tip. With reference to FIG. 8B, the arm 820 is depicted in a cutting condition with the arm distal end portion 823 extending radially outward from the longitudinal axis and the distal end portion 803 to define a hook. As described above, the at least one arm 820 is configured to extend through an orifice defined between native heart valve leaflets attached together by a fixation device to position the cutter 830 against one of the native heart valve leaflets adjacent the fixation device.

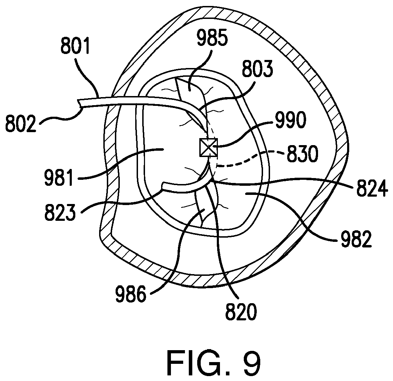

In accordance with an aspect of the disclosed subject matter, the native heart valve can be a native mitral valve. FIG. 9, depicts a cross-sectional view of a native mitral valve taken along the native mitral valve annulus looking down from the left atrium. The native mitral valve has a fixation device 990 attaching the native heart valve leaflets 981, 982 together. The exemplary system 800 is positioned within the native mitral valve with the at least one arm 820 in a cutting condition. In accordance with an aspect of the disclosed subject matter, the at least one arm 820 can be positioned around native heart valve leaflet 981 with the distal end portion 803 and the arm distal end portion 824 extending through opposing orifices 985, 986 on either side of the native heart valve leaflet 981. As described above, the opposing orifices 985, 986 are defined between the native heart valve leaflets 981, 982 attached together by fixation device 990.

Positioning the arm around the native heart valve leaflet 981 can position the cutter 830 against the native heart valve leaflet. For example and as embodied herein, the cutter 830 can be positioned along the native heart valve leaflet 981 on a ventricular side thereof adjacent to the fixation device 990. As described further herein, controlling the position of the cutter 830 relative to the fixation device 990 can be desirable when separating the native heart valve leaflets. For example, cutting the native heart valve leaflet tissue in close proximity to the fixation device 990 can minimize the amount of native heart valve leaflet tissue extending into the ventricle after separation of the native heart valve leaflets. The position of the cutter can be controlled by, for example, steering the elongate shaft 801 to the desired location. Additionally, the diameter of the arm 820 can be selected to position the cutter 830 along the native heart valve leaflet 981 at a desired distance from the fixation device 990. For example, the cutter 830 can extend along a centerline of the arm 820, and the outer surface of the arm 820 can be guided against the fixation device on the ventricular side of the native mitral valve. With the outer surface of the arm 820 positioned against the fixation device 990, the cutter 830 can be positioned against the ventricular side of the native heart valve leaflet 981 at a distance 995 corresponding to the radius of the elongate shaft 801.

The disclosed subject matter further includes methods for separating native heart valve leaflets attached together by a fixation device. Methods in accordance with the disclosed subject matter include delivering a distal end portion a system for separating native heart valve leaflets to a native heart valve. The system for separating native heart valve leaflets can have any of the features described herein. Methods in accordance with the disclosed subject matter further include extending at least one arm of the system through an orifice defined between native heart valve leaflets attached together by a fixation device and positioning the cutter against one of the native heart valve leaflets adjacent the fixation device. Methods in accordance with the disclosed subject matter further include cutting the native heart valve leaflet with the cutter to separate the native heart valve leaflets.

With continued reference to FIG. 9, the exemplary system 800 can be used in accordance with an aspect of the disclosed subject matter to separate native mitral valve leaflets attached together by a fixation device. As embodied herein, the arm distal end portion 823 and arm length 824 can be longitudinally aligned with the elongate shaft 801 for delivery to and extension through the native heart valve. For example, the native mitral valve can be accessed transeptally by crossing the atrial wall above the native mitral valve, and the elongate shaft 801 and arm 820 can be delivered through orifice 985 from the atrial side of the native mitral valve with the arm 820 aligned with the longitudinal axis in a delivery condition. The system 800 can be delivered with a transseptal sheath or introducer catheter for passing through the septum, or alternatively, the system 800 can be independently passed through the septum.

In accordance with an aspect of the disclosed subject matter, extending the arm 820 can include deploying the arm 820 to a cutting condition, the arm 820 in the cutting condition extending radially outward from the longitudinal axis and the distal end portion 803 to define a hook with the distal portion 823 and the arm distal end portion 803 extending through opposing orifices 986, 985 on either side of the native heart valve leaflet 981. As noted above, positioning the arm around the native heart valve leaflet 981 can position the cutter 830 against the native heart valve leaflet. As embodied herein, the cutter 830 can be positioned along the native heart valve leaflet 981 on a ventricular side thereof with the arm 820 in the cutting condition. Cutter 830 is depicted in broken line in FIG. 9 for purpose of illustration and understanding.

Methods in accordance with the disclosed subject matter further include cutting the native heart valve leaflet 981 with the cutter 830 to separate the native heart valve leaflets. As embodied herein, cutting the native heart valve leaflet 981 can include retracting the elongate shaft along the longitudinal axis with the cutter 830 positioned along the native heart valve leaflet 981 to pull the cutter 830 through the native heart valve leaflet 981. For example, the cutter 830 can comprise a stainless steel wire as described above, and the wire can cut through the leaflet 981 as the elongate shaft is retracted along the longitudinal axis. The construction of the elongate shaft 801 and arm 820 can be selected to provide the desired rigidity such that the cutter 830 is maintained in tension as it cuts through the leaflet 981.. Additionally or alternatively, and as embodied herein, the cutter can also be configured to apply RF energy to the native heart valve leaflet 981 to cut the native heart valve leaflet tissue.

As described above, separation of native heart valve leaflets attached by a fixation device can be desirable for a number of clinical reasons. For example, sometimes after a fixation device is installed mitral valve regurgitation can still exist, or can arise again. In some cases, it can be desirable to remove the fixation device to allow for implantation of a replacement heart valve. Accordingly, and in accordance with another aspect of the disclosed subject matter, after separating the native heart valve leaflets as described herein, a prosthetic heart valve can be installed at the native heart valve. The prosthetic heart valve can be delivered to and installed at the native heart valve using techniques known in the art. For example, the prosthetic heart valve can be delivered transapically, or alternatively, can be delivered transvascularly, including transeptally. Examples of prosthetic heart valves and methods for delivering prosthetic heart valves are described in U.S. Patent Numbers. 10,751,173, 10,470,881, 9,439,757, and 8,870,948, the content of each of which is hereby incorporated by reference in its entirety.

In accordance with an aspect of the disclosed subject matter, the anterior mitral valve leaflet 981 can be cut to separate the native heart valve leaflets. Cutting the anterior mitral valve leaflet can facilitate subsequent operations on the native mitral valve, such as valve replacement procedures. For example, after cutting the anterior mitral valve leaflet 981, the fixation device 990 can remain attached to the posterior leaflet 982, which can be desirable, including for reducing the risk of left ventricular outflow tract obstruction ("LVOTO"). For example, installation of a prosthetic valve at the native mitral valve after separation of the native heart leaflets 981, 982 can trap the fixation device 990 and native posterior leaflet 982 against the ventricular wall of the heart away from the aortic valve and left ventricular outflow tract, which can reduce the risk of LVOTO.

Likewise, reducing the amount of anterior mitral valve leaflet 981 tissue that remains attached to the fixation device 990 can also be desirable. As noted above, when cutting the anterior mitral valve leaflet 981, a portion of the anterior mitral valve leaflet 981can remain attached to the fixation device 990 and the posterior mitral valve leaflet 982. The portion of anterior mitral valve leaflet 981 tissue attached to the fixation device 990 after valve separation can potentially compromise the effectiveness of subsequent valve replacement procedures. As noted above, and in accordance with an aspect of the disclosed subject matter, the amount of anterior mitral valve leaflet 981 that remains attached to the fixation device 990 can be reduced by cutting the anterior mitral valve leaflet 981 in close proximity to the fixation device 990.

Methods in accordance with the disclosed subject matter can include additional features. For example, methods can include weakening native heart valve leaflet tissue before cutting the native heart valve. Weakening the native heart valve leaflet tissue can help ensure that the native heart valve leaflet tissue cuts or tears at the desired location. Any suitable means for weakening the native anterior leaflet tissue can be used. For example, one or more of a needle, blade, RF energy, and hydrogen peroxide can be applied to the native heart valve leaflet tissue to weaken the tissue prior to cutting.

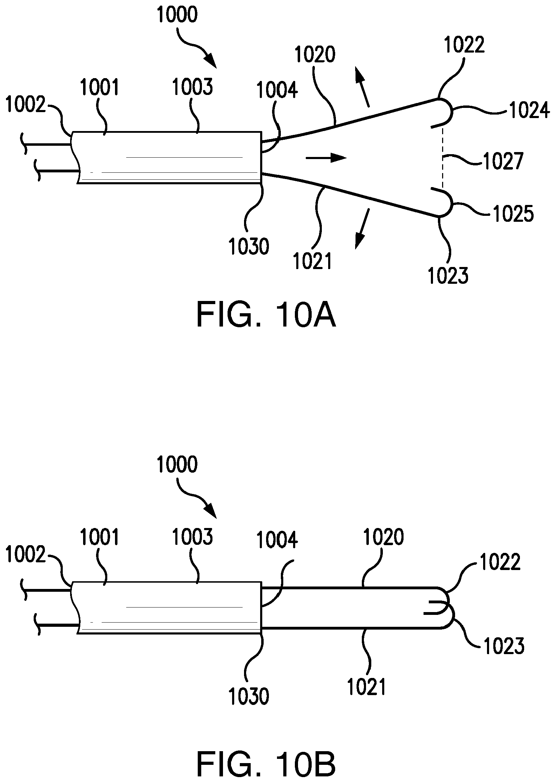

With reference to FIGs. 10-13, systems for separating native heart valve leaflets attached together by a fixation device in accordance with the disclosed subject matter can include additional aspects. For example, and with reference to FIGs. 10A-11B, exemplary system 1000 includes an elongate shaft 1001 having a proximal end portion 1002, a distal end portion 1003, and a longitudinal axis extending therebetween. The elongate shaft 1001 is configured for transvascular delivery of the distal end portion 1003 to a native heart valve. Arms 1020 and 1021 extend from the distal end portion 1003, and a cutter 1030 is disposed along the distal end portion 1003. The arms 1020 and 1021 are configured to extend through orifices defined between native heart valve leaflets attached together by a fixation device to position the cutter 1030 against one of the native heart valve leaflets adjacent the fixation device. The cutter 1030 is configured to cut through native heart valve leaflet tissue.

In accordance with an aspect of the disclosed subject matter, the distal end portion 1003 of the elongate shaft 1001 can include at least one lumen 1004 defined therein, and the arms 1020 and 1021 can be deployable from the lumen 1004 between a retracted position and an extended position. For example, the arms 1020 and 1021 can be disposed within the lumen 1004 for transvascular delivery to the native heart valve and deployed to the extended position at the native heart valve. With reference to FIG. 10A, the arms 1020 and 1021 are shown deploying from the lumen 1004 from the retracted position to the extended position.

As embodied herein, the system 1000 includes two opposing arms 1020 and 1021. The opposing arms 1020 and 1021 can be configured to spread away from one another as the arms 1020 and 1021 deploy from the retracted position to the extended position. For purpose of example, the arms can be made of a shape memory material such as nitinol or stainless steel and configured to bias outwards when not restricted by the walls of the lumen 1004. As embodied herein, a distance 1027 can be defined between distal ends 1024, 1025 of the arms 1020 and 1021 when the arms are in the extended position. The unrestrained shape of the arms can be selected to achieve the desired distance 1027. The opposing arms 1020 and 1021 in the extended position are configured to extend through opposing orifices on either side of one of the native heart valve leaflets. As further embodied herein, the opposing arms 1020, 1021 can be retracted from the extended position back into the lumen 1004 towards a retracted position. With reference to FIG. 10B, the opposing arms 1020, 1021 can be configured to approach one another as the arms 1020, 1021 retract back into the lumen.

The exemplary system 1000 further includes a cutter 1030 configured to cut through native heart valve leaflet tissue. In accordance with an aspect of the disclosed subject matter, a distal end of the elongate shaft 1001 can define the cutter 1030. For example the distal end portion 1003 of the elongate shaft can include a sharp edge, such as a point, or blade, and the arms 1020, 1021 can be used to pull a native heart valve leaflet against the cutter 1030 at the distal end portion 1003 of the elongate shaft as described further below. As embodied herein, the arms 1020, 1021 in the extended position can define hooks 1022, 1023, the hooks configured to be positioned around one of the native heart valve leaflets and to draw the native heart valve leaflet against the cutter 1030 as the arms 1020, 1021 are retracted towards the retracted position to cut through native heart valve leaflet tissue.

Additionally or alternatively, one or both of the arms 1020, 1021 can define the cutter 1030. For example, one or both of the arms can include a sharpened edge. Additionally or alternatively, and as embodied herein, one or both of the arms 1020, 1021 can be constructed of a conductive material and configured to transmit RF energy to cut native heart valve leaflet tissue as described above. Although the exemplary system 1000 includes two arms 1020, 1021, it is to be understood that systems in accordance with the disclosed subject matter can include a single arm, or more than two arms.

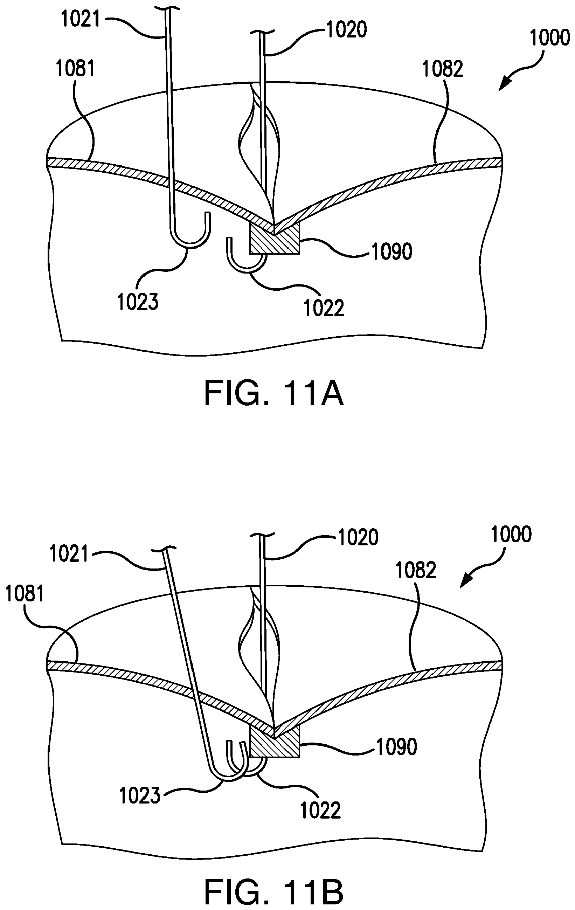

With reference to FIGs. 11A and 11B, exemplary system 1000 can be used in accordance with methods of the disclosed subject matter to separate native heart valve leaflets attached together by a fixation device. As embodied herein, arms 1020, 1021 can be extended through orifices defined between native heart valve leaflets 1081, 1082 attached together by a fixation device 1090. In accordance with an aspect of the disclosed subject matter, extending arms 1020, 1021 through the orifices can include deploying the arms 1020, 1021 from a retracted position within the lumen 1004 towards an extended position. With reference to FIG. 11A, deploying the arms 1020, 1021 can include positioning the arms 1020, 1021 through opposing orifices on either side of the native anterior mitral valve leaflet 1081. As embodied herein, the arms 1020, 1021 in the extended position can define hooks 1022, 1023, and extending the arms through the opposing orifices can further include positioning the hooks 1022, 1023 around the native anterior mitral valve leaflet 1081.

In accordance with an aspect of the disclosed subject matter, arms 1020, 1021 can be retracted into lumen 1004 within the elongate shaft 1001 to cut the native heart valve leaflet 1081. As embodied herein and with reference to FIG. 11B, the arms 1020, 1021 can be configured to approach one another as the arms 1020, 1021 retract to sandwich the native heart valve leaflet 1081 therebetween within the hooks 1022, 1023. For example, as the arms 1020, 1021 retract, the diameter of the lumen 1004 can restrict the arms and cause the arms 1020, 1021 to approach one another. As the arms 1020, 1021 further retract, the arms 1020, 1021 can draw the native heart valve leaflet 1081 against the cutter 1031 to cut the native heart valve leaflet 1081. Additionally or alternatively, one or more of the arms 1020, 1021 can apply RF energy to the native heart valve leaflet 1081 to cut the native heart valve leaflet, as noted above.

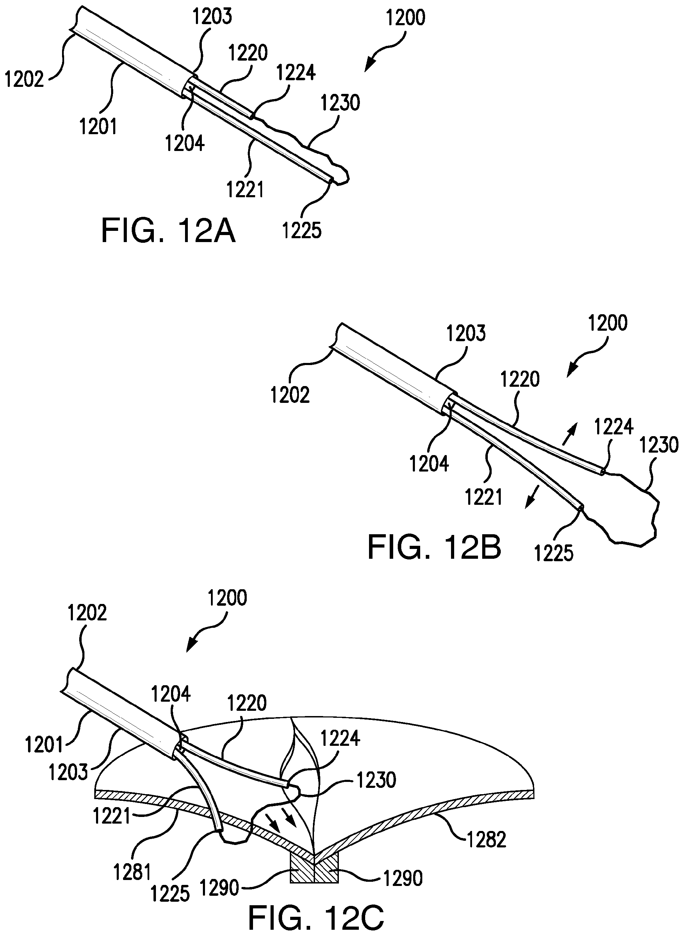

FIGs. 12A-12C depict exemplary system 1200 in accordance with another aspect of the disclosed subject matter. The system 1200 includes an elongate shaft 1201 having a proximal end portion 1202, a distal end portion 1203, and a longitudinal axis extending therebetween. The elongate shaft 1201 is configured for transvascular delivery of the distal end portion 1203 to a native heart valve. Arms 1220 and 1221 extend from the distal end portion 1203. Cutter 1230 is disposed between arms 1220 and 1221. The arms 1220 and 1221 are configured to extend through orifices defined between native heart valve leaflets attached together by a fixation device to position the cutter 1230 against one of the native heart valve leaflets adjacent the fixation device. The cutter 1230 is configured to cut through native heart valve leaflet tissue.

As described above, the distal end portion 1203 can include a lumen 1204 defined therein, and arms 1220, 1221 and cutter 1230 can be deployable from the lumen between a retracted position and an extended position. For purpose of example and as embodied herein, the cutter 1230 and arms 1220, 1221 can be disposed within the lumen 1204 for delivery of the system to the native heart valve and deployed to the extended position at the native heart valve. With reference to FIG. 12A, the arm distal ends 1224, 1225 can be offset along the longitudinal axis within the lumen 1204 to align the cutter 1230 within the lumen 1204 for delivery to the native heart valve.

As depicted in FIG. 12B, the arms 1220, 1221 can be configured to bias or extend away from one another as the arms deploy out of the lumen 1204 toward the extended position. The arms 1220, 1221 in the extended position can define an opposing arm distance between the arm distal ends 1224, 1225. As embodied herein, the arms 1220, 1221 can be comprised of a rigid material with each arm configured to withstand a force or pressure applied at the distal ends 1224, 1225 thereof without buckling. For example the arms can be constructed of pre-shaped nitinol or stainless steel material configured to provide the desired bias and rigidity. The construction and rigidity of the arms 1220, 1221 can be selected to maintain a desired pressure on the cutter 1230, as described further herein. While the rigid plurality of arms and the bridging flexible cable or monofilament wire are joined and all conductive to electrical currents, the arms can be insulated to minimize discharge to adjacent tissue and to focus monopolar RF energy along the flexible bridging element. The bridging element can lack insulation or can have selective insulation to focus RF discharge at the apex or center. The more flexible bridging element can present itself as a funnel shape to receive leaflets into the apex of the funnel, and when axial translation or pressure is applied between the non-insulated bridging element and the leaflet tissue targeted for cutting, the device can be energized at the proximal end to cut at the distal end of the device.

In accordance with an aspect of the disclosed subject matter, the cutter 1230 can extend between arms 1220, 1221, and the arms 1220, 1221 can be configured to extend through opposing orifices defined between native heart valve leaflets attached together by a fixation device. The arms 1220, 1221 can be further configured to position the cutter 1230 against one of the native heart valve leaflets adjacent the fixation device. As embodied herein, the cutter 1230 can be a wire extending between distal ends 1224, 1225 of arms 1220, 1221. The opposing arm distance and length of the cutter 1230 can be selected to apply a desired amount of tension to the cutter 1230 when the arms 1220, 1221 are in the extended position. For purpose of example, the the opposing arm distance can be between approximately 5mm and approximately 15mm.

The tensioned cutter 1230 can be positioned along a native heart valve leaflet and the elongate shaft 1201 can be advanced to guide the cutter 1230 through the native heart valve leaflet tissue and separate the native heart valve leaflets. As described above, the cutter 1230 can be constructed of a stainless steel wire and the wire gauge can be selected such that the tensioned wire cuts through the native heart valve leaflet tissue as it is advanced. Additionally or alternatively, monopolar or bipolar RF energy can be applied to the cutter 1230 to cut through the native heart valve leaflet tissue. The cutter 1230 can be positioned along the native heart valve leaflet in close proximity to the fixation device to minimize the amount of heart valve leaflet that remains attached in the fixation device after cutting as described above.

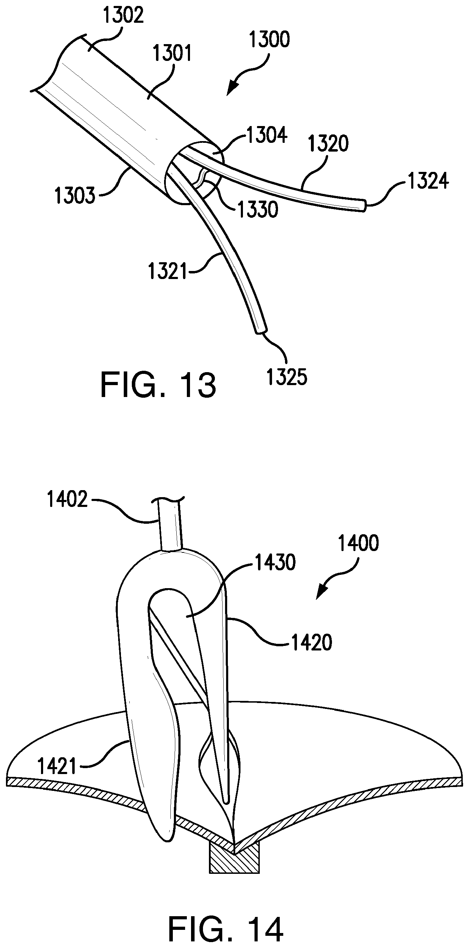

In accordance with another aspect of the disclosed subject matter, and with reference to the exemplary system 1330 depicted in FIG. 13A, the cutter 1330 can extend between arms 1320 and 1321 proximal to the distal arm ends 1324, 1325. System 1300 includes an elongate shaft 1301 having a proximal end portion 1302, a distal end portion 1303, and a longitudinal axis extending therebetween. The elongate shaft 1301 is configured for transvascular delivery of the distal end portion 1303 to a native heart valve. Arms 1320 and 1321 extend from the distal end portion 1303, and a cutter 1330 is disposed along the distal end portion 1303. The arms 1320 and 1321 are configured to extend through orifices defined between native heart valve leaflets attached together by a fixation device to position the cutter 1330 against one of the native heart valve leaflets adjacent the fixation device. The cutter 1330 is configured to cut through native heart valve leaflet tissue.

As embodied herein, the cutter 1330 can extend between arms 1320, 1321 at a location between the distal end portion 1303 and the arm distal ends 1324, 1325. The location of the cutter 1330 can be selected to achieve desired performance characteristics. For example with the cutter 1330 proximal to the distal ends 1324, 1325, the distal ends 1324, 1325 can be used to guide or funnel the native heart valve leaflet tissue towards the cutter 1330 as the arms 1320, 1321 extend through orifices on either side of the native heart valve leaflet. As described above, the cutter 1330 can include, for example, a mechanical blade, or thin gauged wire. Additionally or alternatively, the cutter 1330 can be configured to apply RF energy to cut through native heart valve leaflet tissue as described above. In accordance with another aspect of the disclosed subject matter, one or more of the arms 1320, 1321 can be configured to apply RF energy to cut through native heart valve leaflet tissue.

With reference to FIG. 14, systems in accordance with another aspect of the disclosed subject matter can include fixed opposing arms extending from the distal end portion of the elongate shaft to define a funnel shape. Exemplary system, 1400 includes an elongate shaft 1401 having a proximal end portion 1402, a distal end portion 1403, and a longitudinal axis extending therebetween. The elongate shaft 1401 is configured for transvascular delivery of the distal end portion 1403 to a native heart valve. Arms 1420 and 1421 extend from the distal end portion 1403, and a cutter 1430 is disposed along the arm 1420. The arms 1420 and 1421 are configured to extend through orifices defined between native heart valve leaflets attached together by a fixation device to position the cutter 1430 against one of the native heart valve leaflets adjacent the fixation device. The cutter 1430 is configured to cut through native heart valve leaflet tissue.as described above. As described above, the 1420, 1430 arms can be configured to guide native heart valve leaflet tissue towards a cutter as the opposing arms are extended through opposing orifices on either side of a native heart valve leaflet.

The disclosed subject matter further includes a kit for treating a native heart valve having leaflets attached together by a fixation device. The kit includes a system for separating native heart valve leaflets attached together by a fixation device. The system can include the features described above. The kit also includes a prosthetic heart valve.

### Exemplary Embodiments:

A1. A system for separating native heart valve leaflets attached together by a fixation device, the system comprising an elongate shaft having a proximal end portion, a distal end portion and a longitudinal axis extending therebetween, the elongate shaft configured for transvascular delivery of the distal end portion to a native heart valve. The system further comprises at least one arm extending from the distal end portion and a cutter disposed along at least one of the distal end portion and the at least one arm, wherein the at least one arm is configured to extend through an orifice defined between native heart valve leaflets attached together by a fixation device to position the cutter against one of the native heart valve leaflets adjacent the fixation device, the cutter configured to cut through native heart valve leaflet tissue.

A2. The system of A1, wherein the elongate shaft includes a steering mechanism adapted to bend at least a portion of the elongate shaft in at least a first reference plane.

A3. The system of A2, wherein the steering mechanism is adapted to bend at least a portion of the elongate shaft in a second reference plane, the second reference plane being generally perpendicular to the first reference plane.

A4. The system of A2-A3, wherein the steering mechanism includes at least one cable extending the length of the elongate shaft.

A5. The system of claim A1-A4, wherein the at least one arm includes an arm distal end portion and an arm length extending between the distal end portion and the arm distal end portion, the at least one arm deployable between a delivery condition and a cutting condition, the at least one arm in the delivery condition extending along the longitudinal axis, and the at least one arm in the cutting condition extending radially outward from the longitudinal axis and the distal end portion to define a hook, the at least one arm configured to be positioned around one of the native heart valve leaflets with the distal end portion and the arm distal end portion extending through opposing orifices on either side thereof defined between the native heart valve leaflets attached together by a fixation device.

A6. The system of A5, wherein the cutter extends between the distal end portion and the arm distal end portion.

A7. The system of A5-A6, wherein a slot is defined in an outer wall of the at least one arm, the slot extending between the distal end portion and the arm distal end portion, and wherein the cutter is configured to be deployed from the slot to extend away from an apex of the hook in the cutting condition.

A8. The system of claim A7, wherein a cutter height is defined between the cutter and the apex of the hook in the cutting condition, the cutter height being between approximately 5mm and approximately 10mm.

A9. The system of A4-A8, wherein the cutter is radially offset from the at least one cable when the elongate shaft is viewed in transverse cross section.

A10. The system of A1-A9, wherein the cutter includes a wire.

A11. The system of A5-A10, wherein the cutter is affixed to the arm distal end portion, and further wherein the cutter is configured to transition the at least one arm from the delivery condition to the cutting condition upon application of tension thereto.

A12. The system of A5-A11, wherein a hook width is defined across the hook between the distal portion and arm distal end portion in the cutting condition, the hook width being between approximately 5mm and approximately 15mm.

A13. The system of A1-A12, wherein the cutter includes a conductive pad extending along the at least one arm.

A14. The system of A13, wherein the conductive pad is adhered to an outer wall of the at least one arm.

A15. The system of A1-A14, wherein the cutter is conductive and is configured to be energized with RF energy to cut through native heart valve leaflet tissue.

A16. The system of A15, wherein the RF energy is monopolar.

A17. The system of A15, wherein the RF energy is bipolar and the system includes a current return path.

A18. The system of A1-A17, wherein the distal end portion includes at least one lumen defined therein, the at least one arm deployable from the lumen between a retracted position and an extended position.

A19. The system of A1-A18, wherein the at least on arm comprises one or more of stainless steel, and nitinol.

A20. The system of A18-A19, wherein the at least one arm in the extended position defines a hook, the hook configured to be positioned around one of the native heart valve leaflets and to draw the native heart valve leaflet against the cutter as the at least one arm is retracted towards the retracted position to cut through native heart valve leaflet tissue.

A.21. The system of A1-A20, wherein the cutter is disposed at the distal portion of the elongate shaft.

A22. The system of A1-A21, wherein the cutter comprises a blade.

A23. The system of A1-A22, wherein the at least one arm includes the cutter.

A24. The system of A1-A23, wherein the at least one arm includes two opposing arms.

A25. The system of A24, wherein the opposing arms are configured to spread away from one another as the opposing arms deploy from the retracted position toward the extended position, the opposing arms in the extended position configured to extend through opposing orifices on either side of one of the native heart valve leaflets defined between the native heart valve leaflets attached together by a fixation device, the opposing arms configured to approach one another as the opposing arms are retracted towards the retracted position to trap the native heart valve leaflet therebetween.

A26. The system of A25, wherein opposing arm distal ends of the opposing arms in the extended position define an opposing arm distance therebetween, the opposing arm distance being between approximately 5mm and approximately 15mm.

A27. The system of A24-A26, wherein the cutter includes a wire extending between the opposing arms.

A28. The system of A27, wherein the wire extends between respective opposing arm distal ends.

A29. The system of A24-A28, wherein the opposing arms are rigid.

A30. The system of A24-A29, wherein the cutter is disposed along at least one of the opposing arms.

In addition to the specific embodiments claimed below, the disclosed subject matter is also directed to other embodiments having any other possible combination of the dependent features claimed below and those disclosed above. As such, the particular features presented in the dependent claims and disclosed above can be combined with each other in other manners within the scope of the disclosed subject matter such that the disclosed subject matter should be recognized as also specifically directed to other embodiments having any other possible combinations. Thus, the foregoing description of specific embodiments of the disclosed subject matter has been presented for purposes of illustration and description. It is not intended to be exhaustive or to limit the disclosed subject matter to those embodiments disclosed.

It will be apparent to those skilled in the art that various modifications and variations can be made in the method and system of the disclosed subject matter without departing from the spirit or scope of the disclosed subject matter. Thus, it is intended that the disclosed subject matter include modifications and variations that are within the scope of the appended claims and their equivalents.

## Claims

1. A system for separating native heart valve leaflets attached together by a fixation device, comprising:
an elongate shaft having a proximal end portion, a distal end portion and a longitudinal axis extending therebetween, the elongate shaft configured for transvascular delivery of the distal end portion to a native heart valve;
at least one arm extending from the distal end portion; and
a cutter disposed along at least one of the distal end portion and the at least one arm;
wherein the at least one arm is configured to extend through an orifice defined between native heart valve leaflets attached together by a fixation device to position the cutter against one of the native heart valve leaflets adjacent the fixation device, the cutter configured to cut through native heart valve leaflet tissue.

2. The system of claim 1, wherein the elongate shaft includes a steering mechanism adapted to bend at least a portion of the elongate shaft in at least a first reference plane, and optionally, wherein the steering mechanism is adapted to bend at least a portion of the elongate shaft in a second reference plane, the second reference plane being generally perpendicular to the first reference plane, and optionally, wherein the steering mechanism includes at least one cable extending the length of the elongate shaft.

3. The system of claim 2, wherein the cutter is radially offset from the at least one cable when the elongate shaft is viewed in transverse cross section.

4. The system of any one of claims 1-3, wherein the at least one arm includes an arm distal end portion and an arm length extending between the distal end portion and the arm distal end portion, the at least one arm deployable between a delivery condition and a cutting condition, the at least one arm in the delivery condition extending along the longitudinal axis, and the at least one arm in the cutting condition extending radially outward from the longitudinal axis and the distal end portion to define a hook, the at least one arm configured to be positioned around one of the native heart valve leaflets with the distal end portion and the arm distal end portion extending through opposing orifices on either side thereof defined between the native heart valve leaflets attached together by a fixation device, and optionally, wherein the cutter extends between the distal end portion and the arm distal end portion.

5. The system of claim 4, wherein a slot is defined in an outer wall of the at least one arm, the slot extending between the distal end portion and the arm distal end portion, and wherein the cutter is configured to be deployed from the slot to extend away from an apex of the hook in the cutting condition, and optionally, wherein a cutter height is defined between the cutter and the apex of the hook in the cutting condition, the cutter height being between approximately 5mm and approximately 10mm, and optionally, wherein a hook width is defined across the hook between the distal portion and arm distal end portion in the cutting condition, the hook width being between approximately 5mm and approximately 15mm.

6. The system of any one of claims 4-5, wherein the cutter is affixed to the arm distal end portion, and further wherein the cutter is configured to transition the at least one arm from the delivery condition to the cutting condition upon application of tension thereto.

7. The system of any one of claims 1-6, wherein the distal end portion includes at least one lumen defined therein, the at least one arm deployable from the lumen between a retracted position and an extended position.

8. The system of claim 7, wherein the at least one arm in the extended position defines a hook, the hook configured to be positioned around one of the native heart valve leaflets and to draw the native heart valve leaflet against the cutter as the at least one arm is retracted towards the retracted position to cut through native heart valve leaflet tissue.

9. The system of any one of claims 1-8, wherein the at least one arm includes two opposing arms, and optionally, wherein the opposing arms are rigid.

10. The system of claim 9, wherein the opposing arms are configured to spread away from one another as the opposing arms deploy from the retracted position toward the extended position, the opposing arms in the extended position configured to extend through opposing orifices on either side of one of the native heart valve leaflets defined between the native heart valve leaflets attached together by a fixation device, the opposing arms configured to approach one another as the opposing arms are retracted towards the retracted position to trap the native heart valve leaflet therebetween, and optionally, wherein opposing arm distal ends of the opposing arms in the extended position define an opposing arm distance therebetween, the opposing arm distance being between approximately 5mm and approximately 15mm.

11. The system of any one of claims 1-10, wherein the cutter includes at least one of a wire, a conductive pad, and a blade, and optionally wherein the cutter is conductive and is configured to be energized with RF energy to cut through native heart valve leaflet tissue, optionally wherein the RF energy is monopolar, and optionally wherein the RF energy is bipolar and the system includes a current return path.

12. The system of any one of claims 9-11, wherein the cutter includes a conductive pad extending along the at least one arm, and optionally, wherein the conductive pad is adhered to an outer wall of the at least one arm.

13. The system of any one of claims 9-12, wherein the cutter includes a wire extending between the opposing arms, and optionally wherein the wire extends between respective opposing arm distal ends.

14. The system of any one of claims 9-13, wherein the cutter is disposed along at least one of the opposing arms.

15. The system of any one of claims 1-14, wherein the cutter is disposed at the distal portion of the elongate shaft, and optionally wherein the at least one arm includes the cutter.
